# EUROPEAN PATENT SPECIFICATION

(11) **EP 1 313 495 B1**
(45) Date of publication and mention of the grant of the patent: **24.11.2004**
(21) Application number: 01961557.4
(22) Date of filing: 31.08.2001
(51) Int. Cl.: A61K 35/78, A61K 35/64, A61P 15/12, A61K 31/355

(54) **USE OF A COMPOSITION COMPRISING AN EXTRACT OF POLLEN FOR THE TREATMENT OF HORMONALLY-RELATED DISORDERS**
VERWENDUNG EINER POLLENEXTRAKT-HALTIGEN VERBINDUNG ZUR BEHANDLUNG HORMON-ASSOZIIERTER ERKRANKUNGEN
UTILISATION D'UNE COMPOSITION CONTENANT UN EXTRAIT DE POLLEN POUR TRAITER DES TROUBLES D'ORDRE HORMONAUX

(30) Priority: 01.09.2000 SE 0003092
(43) Date of publication of application: 28.05.2003
(73) Proprietor: Natumin Pharma AB, 56151 Huskvarna (SE)
(72) Inventor: WINTER, Kaj, DK-2100 Copenhagen (DK); HEDMAN, Christer, S-435 37 Mölnlycke (SE); KÄRNERUD, Lars, S-560 27 Tenhult (SE)
(74) Representative: Allee, Harriet Eva Charlotta
(86) International application number: PCT/SE2001/001851
(87) International publication number: WO 2002/017944

(56) References cited:
- EP-A1- 1 057 483
- US-A- 5 569 459
- 'Menopause treatment options', [Online] XP002906802 Retrieved from the Internet: <URL:http://www.menopause.org/mgremedies.ht m> [retrieved on 2001-04-20]
- 'Femal - Nature help for PMS', [Online] XP002906801 Retrieved from the Internet: <URL:http://www.sisuhealth.com/products/Fem al.html> [retrieved on 2001-04-19]

## Description

### FIELD OF THE INVENTION

The invention, relates to the use of a composition comprising, as active ingredients, a water- and/or fat-soluble cytosolic extract of pollen and optionally pistils for the manufacturing of a medicament for the treatment of disorders relating to normal hormonal change in women in peri- and post-menopausal age.

### BACKGROUND OF THE INVENTION

Menopause, which is caused by a lowering of the production of female sex hormones at the age around 50, can to many women generate disorders such as hot flushes, attacks of sweating, muscle and possibly joint pain, sleep disturbances, dysphoria, nervousness, mood swings, headache, palpitations (enhanced frequency of heart rate), dry mucous membranes and pain during intercourse, urinary disturbances such as stress incontinence, frequent passing water and pain/irritability of the bladder and urethra during the process of passing water etc. All these disorders reflect age related hormonal changes which hitherto only have been alleviated effectively by the administration of female sex hormones like estrogen and the like.

Four out of five women have disturbing menopause disorders for at least one year and 25% of women have menopause disorders for more than 5 years. Half of all women have severe disorders and a population of 5 million inhabitants will constantly include about 200.000 women in the period of life where menopause trouble is disrupting their life.

Over the years different treatment options have been suggested to be effective for the treatment of the above-mentioned disorders. However few have shown a consistent efficacy on the majority thereof. The pharmacological treatments that have been tried include hormonal treatment as well as dietary interventions with e.g. vitamin and mineral supplementation, and natural products. Many of the compounds used for the treatment are limited due to adverse effects. Therefore there remains a great need for safe compounds with a consistent efficacy.

A composition comprising an extract of combined pollen and pistils combined with a pollen grain extract, Royal Jelly and Vitamin E has been sold by Natumin Pharma AB, Kungsängsvägen 27, 561 56 Huskvarna, Sweden, for the treatment of PreMenstrual Syndrome (PMS).

### SUMMARY OF THE INVENTION

The present inventors now have found that the administration of the present active composition, previously known only as a remedy to alleviate disorders related to PMS, provides a surprising and unexpected relief of disorders obstructing the normal life of women in the peri-and post-menopause without negative adverse effects.

The present inventors thus found an unexpected beneficial effect on disorders in relation to normal hormone variations in women of peri- and post-menopausal age, obtained by the administering of a composition comprising, as active ingredients, a water- and/or fat-soluble cytosolic extract of pollen and optionally pistils, optionally in combination with Royal Jelly and Vitamin E.

The invention is based on this discovery.

The present invention provides a composition comprising, as active ingredients, a water- and/ or fat-soluble cytosolic extract of pollen and optionally pistils optionally combined with Royal Jelly and Vitamin E for manufacturing of a medicament for the treatment of disorders in relation to normal hormone variations in women of peri- and post-menopausal age.

In particular, the composition of the present invention is effective against such disorders as hot flashes, tendencies of sweating, palpitations, muscle pains, headache, difficulties in passing water, stress incontinence, dysphoria, dry vaginal and mucous membranes, arthralgia, water retention, irritability, and variations in mood.

The scope and preferred embodiments of the invention are as defined in the appended claims.

### DETAILED DESCRIPTION OF THE INVENTION

The water-and/or fat-soluble cytosolic extract of pollen and optionally pistils preferably comprises an extract of pollen and pistils (PI 82) combined with an extract of pollen grain (GC FEM), as specified herein below. Both types of extract may be purchased from Allergon AB, Välingevägen 309, S-262 92 Ängelholm, Sweden, a Pharmacia company.

The pollens and pistils used for manufacturing of PI82 and GC FEM extracts are selected and harvested primarily from plants belonging to the grass family *(Poaceae).* During processing, treatment is performed on the pollen to open and remove the outer cell wall thereof as well as to minimize the risk of allergic pollen reactions.

PI 82 is a cytosolic pollen-pistil extract rich in superoxide dismutase (SOD) mimics. The source of PI 82 is freshly harvested pollen grains and pistils. The pollen and pistils are allowed to react under very specific conditions. In the reaction, the process of fertilization is initiated between pollen and pistils, and the extract comprises the product thereof. Substances obtained in this reaction are SOD mimics, flavonoids, tannins and polyphenols. In vitro studies have shown that the extract has high superoxide dismutase activity and prevents the formation of free radicals. Experiences from double blind patient investigation show that PI 82 protects the body from the negative influence of free radicals. Furthermore, PI 82 improves the red cell function, thus improving oxygen perfusion to different tissues.

GC FEM is a water-soluble cytosolic extract from pollen. The cytoplasm content of the pollen contains, in addition to the above-mentioned substances, a high amount of carbohydrates and protein. Furthermore, carotenoids and traces of estrogen substances are found. GC FEM contains natural bioflavonoids, vitamins, enzymes and trace elements.

Royal Jelly is a product of the processing of various plant materials within the salivary glands of the worker honeybees. It is rich in pantothenic acid (also called Vitamin B₅), further vitamins and sterols. It is preferably included in the composition in lyophilized form, preferably concentrated by drying to a ratio of at least 1:3. Royal Jelly may be purchased from AB Montoil, Box 24150, S-104 51 Stockholm, Sweden.

As a source of Vitamin E, use is preferably made of Dry Vitamin E 50%, Type SD, from F. Hoffmann-La Roche Ltd., CH-4070 Basel, Switzerland. Preferably dl-alphatocopherol is used, but other forms might also be valuable.

Further, common drug processing compounds may be included, such as diluents, glidants, lubricants, disintegrants, flavoring and coloring agents well known to the man skilled in the art of pharmaceutical sciences.

The active ingredients may be included in formulations of any form, such as tablets, powders, granules, and tinctures. They also may be included in foodstuff of different origin, e.g. as functional food. Administration to the woman in need thereof may take place several times a day, such as 1-8 times daily, 1-6 times daily or 1-4 times daily.

A daily dosage may contain from 60 to 960 mg of PI 82; from 18 to 288 mg of GC FEM; and optionally from 2 to 48 mg of Royal Jelly. Vitamin E might be included in an amount of from 5 to 80 mg.

Preferably, a daily dosage may contain from 60 to 480 mg of PI 82; from 20 to 140 mg of GC FEM; optionally from 2 to 30 mg of Royal Jelly; and optionally from 5 to 60 mg/day Vitamin E.

More preferably, a daily dosage may contain from 60 to 360 mg of PI 82, 20 to 80 mg of GC FEM, and optionally from 2 to 15 mg of Royal Jelly and optionally from 10 to 40 mg of Vitamin E.

Most preferably, a daily dosage may contain 240 mg PI 82, 72 mg of GC FEM and optionally 12 mg of Royal Jelly and optionally 20 mg of Vitamin E.

It should be noted that, unless no statement to the contrary is made, all amounts of Royal Jelly quoted herein refer to freeze-died, i.e. lyophilized, Royal Jelly. The weight of Royal Jelly before freeze-drying is about at least three times higher, due to the water content of the same.

It furthermore should be noted that all the amounts of Vitamin E quoted herein refer to d-alpha-tocopherol, unless no statement to the contrary is made.

The remedy comprising the active ingredients should be administered to the woman in need thereof daily during at least one month, preferably at least two months.

Other excipients are included in amounts well known to any one skilled in the art of pharmaceutical sciences.

The mode of action is with present scientific knowledge not known. As the product contains three different natural constituents, each with a theoretically contributing effect, the combination of these may be a reason for the observed effect. The constituents PI 82 and GC FEM contain SOD mimics such as flavonoids, tannins and polyphenols. These SOD mimics have an effect on free radical formation, which may be a factor involved in redistribution of fluids, including edema, seen under stress situation. An improved oxygen perfusion may also be a contributing effect.

Vitamin E is added to the composition as an active ingredient or as an antioxidant and stabilizer.

### Test of the effects on disorders relating to normal variations of the sex hormone pattern of women in the peri-and post-menopause

### Test mode

Ten women with an average age of 50.8 (range from 46 to 55) years were recruited for this open trial. All of them had been diagnosed to suffer from climacteric disorders. The menstruation had ceased for 5 women while being irregular for 5. None of the women were subject to any hormone therapy.

Each of the women obtained one tablet comprising the active ingredients in an amount of 120.0 mg PI 82, 36.0 mg GC FEM, 6.0 mg Royal Jelly and 10.0 mg dl- alpha-tocopheryl acetate, twice daily, morning and evening. The treatment did not affect pulse, or systolic or diastolic blood pressure.

The investigated disorders, all relating to variations in the normal sex hormone pattern of women in the peri-and post-menopause, were menopausal hot flashes, tendencies of sweating, palpitations, sleep disorders, vertigo, muscle pains, headache, difficulties in passing water (pollakiuria), stress incontinence, dysphoria, dry-vaginal and mucous membranes, arthralgia, water retention (edema), irritability, and variations in mood.

Evaluation of the disorder was performed by use of a 10-cm VAS (visual analogous scale). Low values express favourable results. All statistical calculations are Wilcoxon test for matched pairs, if not otherwise stated. The results, illustrating the impact of active compositions on different menopausal disorders, are compiled in Table 1.

**Table 1**

| Mean value (standard deviation). | | | |
|---|---|---|---|
| Menopausal disorders | Without treatment (cm) | Treatment by active comp. 1 month (cm) | Treatment by active comp. 2 months (cm) |
| Hot flushes | 3.08 (2.27) | 2.81 (2.03) | 1.33 (2.00)** |
| Sweating tendencies | 3.94 (2.29) | 2.75 (2.37) | 1.73 (2.54)* |
| Palpitations | 2.66 (2.74) | 1.88 (1.37) | 1.31 (1.66)# |
| Muscle pain | 3.40(3.19) | 2.19 (1.42) | 2.08 (2.40)** |
| Headache | 3.77(2.84) | 2.38 (2.20)*** | 2.53 (1.98)*** |
| Stress incontinence or frequent passing water (pollakiuria) | 1.73 (2.34) | 1.15 (1.44) | 0.72 (0.85)# |
| Dysphoria | 3.41(2.05) | 2.16 (1.27)* | 1.60 (1.22)** |
| Dry vaginal and mucous membranes and/or pain during intercourse | 3.02 (2.88) | 1.36 (1.31)*** | 1.52 (2.32)*** |
| Joint pain | 3.94 (2.82) | 1.91 (1.03) | 1.48 (1.30)** |
| Mood | 3.05 (2.26) | 2.72(1.90) | 1.73(1.15) |
| Edema | 6.04 (2.53) | 4.48 (1.93)* | 3.28 (1.59)** |
| Energy loss | 4.99 (2.15) | 3.82 (1.60)* | 2.72 (2.21)*** |
| Irritability | 3.18 (2.55) | 2.20 (1.78)*** | 1.61(1.24)* |
| Sleep disturbances | 4.28 (3.89) | 3.00 (3.07) | 2.87 (3.09) |
| Mood swings | 3.33 (2.16) | 2.31 (1.07) | 1.81 (1.63)# |
| Oversensitivity | 4.03 (2.06) | 2.70 (2.07)* | 2.01 (1.79)** |

| | | | |
|---|---|---|---|
| # = borderline significance | | | |
| * = p<0.05 | | | |
| ** =p<0.02 | | | |
| *** =p<0.01 Hot flushes: significant (p <0.02) after two months. Sweating tendencies: significant (p <0.05) after two months. Palpitations: more than 50% reduction (borderline significant). Muscle pains: significant (p <0.02) after one month and borderline after two months. Headache: highly significant (p <0.01) after one and two months. Stress incontinence and/or pollalduria: reduction of about 60%, borderline significant. Dysphoria: significant after one and two months (p <0.05 and p <0.02). Dry vaginal or mucous membranes and/or pain during intercourse: significant after one and two months (p <0.01 and p <0.01) >50% reduction. Joint pain: significant after two months (p <0.02) Mood: not significant, however a favourable change of about 40% is obvious. Edema/water retention: reduction of more than 50%, significant after one and two months (p <0.05 and p <0.02). Energy loss: marked enhancement, significant after one and two months (p <0.05 and p <0.01). Irritability: reduced by 50% (p <0.01 and p <0.05). Sleep disturbances show a tendency of effect but is not significant. Mood swings: borderline significant after two months. Oversensitivity: significantly better after one and two months (p <0.05 and p <0.02). | | | |

If all VAS scores were added to a common "overall well-being score" a clearly positive effect is shown for 8 of 10 participants, only two were unchanged. Thus, the improvement is significant (p <0.02).

At a direct inquiry of all ten participants whether they have had any advantages or use of the treatment, 6 of them clearly said, "yes" while four answered "don't know" (p <0.05, Chi square).

### EXAMPLE

Below is given an Example of a tablet used according to the invention.

| Active ingredients: | |
|---|---|
| PI 82 (pollen-pistil extract) | 120.0 mg |
| GC FEM (pollen extract) | 36.0 mg |

| Secondary ingredients: | |
|---|---|
| ROYAL JELLY (freeze dried) | 6.0 mg |
| VITAMIN E 50% | 20.0 mg |
| | |

| Other ingredients: | |
|---|---|
| Microcrystalline cellulose | 87.0 mg |
| Dicalcium phosphate | 87.0 mg |
| Magnesium stearate | 4.0 mg |
| | |
| Uncoated tablet weight | 360.0 mg |
| | |

| Coating: | |
|---|---|
| Shellac | approx. 2.64 mg |
| Talc approx. | 0.36 mg |
| | |
| Total weight | approx. 363.0 mg |

## Claims

1. Use of a composition comprising, as active ingredients, a water- and/or fat-soluble cytosolic extract of pollen and/or a water- and/or fat-soluble cytosolic extract of pistils fertilized by pollen, said pollen and/or pollen and pistils being obtained primarily from plants belonging to the grass family *(Poaceae),* optionally combined with Royal Jelly and Vitamin E for manufacturing of a medicament for the treatment of disorders in relation to normal hormone variations in women of peri- and post-menopausal age.

2. Use according to claim 1, wherein the extract of pollen and pistils is PI 82 and the extract of pollen is GC FEM.

3. Use according to claim 2 wherein the active ingredients are included in the medicament in amounts such as to give a daily dosage of from 60 to 960 mg of PI 82; 18 to 288 mg of GC FEM; and optionally 2 to 48 mg of Royal Jelly and 5 to 80 mg of Vitamin E.

4. Use according to claim 3 wherein the active ingredients are included in the medicament in amounts such as to give a daily dosage of from 60 to 480 mg of PI 82; 20 to 140 mg of GC FEM; and optionally 2 to 30 mg of Royal Jelly and 5 to 60 mg of Vitamin E.

5. Use according to claim 4 wherein the active ingredients are included in the medicament in amounts such as to give a daily dosage of from 60 to 360 mg of PI 82; 20 to 80 mg of GC FEM; and optionally 2 to 15 mg of Royal Jelly and 10 to 40 mg of Vitamin E.

6. Use according to claim 5 wherein the active ingredients are included in the medicament in amounts such as to give a daily dosage of 240 mg of PI 82, 72 mg GC FEM, and optionally 12 mg of Royal Jelly and 20 mg of Vitamin E.

7. Use according to any of the claims 1-6 wherein the extract(s) comprise(s) substance(s) selected from of SOD mimics, flavonoids, tannins, polyphenols, carotenoids, traces of estrogen substances, natural bioflavonoids, vitamins, enzymes and trace elements.

8. Use according to any of the claims 1-7, wherein the daily dosage of the medicament is administered in a single dose or in multiple doses, 1-8 times daily.

9. Use according to any of the claims 1-8, wherein the disorders are selected from hot flashes, tendencies of sweating, palpitations, muscle pains, headache, difficulties in passing water, stress incontinence, dysphoria, dry vaginal and mucous membranes, arthralgia, water retention, irritability, and variations in mood.

## Patentansprüche

1. Verwendung einer Zusammensetzung, umfassend, als aktive Zutaten, einen wasser- und/oder fettlöslichen, cytosolischen Extrakt aus Pollen und/oder einen wasser- und/oder fettlöslichen cytosolischen Extrakt aus durch Pollen befruchteten Stempeln, wobei die Pollen und/oder Pollen und Stempel hauptsächlich aus Pflanzen, die zur Süßgräserfamilie (Poaceae) gehören, erhalten werden, wahlweise mit Gelee Royale und Vitamin E kombiniert, zur Herstellung eines Arzneimittels für die Behandlung von Beschwerden im Zusammenhang mit den normalen hormonellen Schwankungen bei Frauen im peri-menopausalen und post-menopausalen Alter.

2. Verwendung gemäß Anspruch 1, worin der Extrakt aus Pollen und Stempeln PI 82 ist und der Extrakt aus Pollen GC FEM ist.

3. Verwendung gemäß Anspruch 2, worin die aktiven Zutaten im Arzneimittel in solchen Mengen beinhaltet sind, dass sich eine tägliche Dosis von 60 bis 960 mg PI 82, 18 bis 288 mg GC FEM und wahlweise 2 bis 48 mg Gelee Royale und 5 bis 80 mg Vitamin E ergibt.

4. Verwendung gemäß Anspruch 3, worin die aktiven Zutaten im Arzneimittel in solchen Mengen beinhaltet sind, dass sich eine tägliche Dosis von 60 bis 480 mg PI 82, 20 bis 140 mg GC FEM und wahlweise 2 bis 30 mg Gelee Royale und 5 bis 60 mg Vitamin E ergibt

5. Verwendung gemäß Anspruch 4, worin die aktiven Zutaten im Arzneimittel in solchen Mengen beinhaltet sind, dass sich eine tägliche Dosis von 60 bis 360 mg PI 82, 20 bis 80 mg GC FEM und wahlweise 2 bis 15 mg Gelee Royale und 10 bis 40 mg Vitamin E ergibt.

6. Verwendung gemäß Anspruch 5, worin die aktiven Zutaten im Arzneimittel in solchen Mengen beinhaltet sind, dass sich eine tägliche Dosis von 240 mg PI 82, 72 mg GC FEM und wahlweise 12 mg Gelee Royale und 20 mg Vitamin E ergibt.

7. Verwendung gemäß einem der Ansprüche 1 bis 6, worin der/die Extrakt(e) Substanzen umfassen, ausgewählt aus SOD Mimics, Flavonoiden, Tanninen, Polyphenolen, Carotinoiden, Spuren von Östrogensubstanzen, natürlichen Bioflavonoiden, Vitaminen, Enzymen und Spurenelementen.

8. Verwendung gemäß einem der Ansprüche 1 bis 7, worin die tägliche Dosierung des Arzneimittels in einer einzelnen oder mehrfachen Dosis 1 bis 8 Mal pro Tag verabreicht wird.

9. Verwendung gemäß einem der Ansprüche 1 bis 8, worin die Beschwerden ausgewählt sind aus Hitzewallungen, Neigungen zu Schweißausbrüchen, Herzklopfen, Muskelschmerzen, Kopfschmerzen, Schwierigkeiten beim Wasserlassen, Siressinkontinenz, Dysphorie, trockene Vagina- und Schleimhäute, Gelenkschmerzen (Arthralgie), Wasseransammlung, Reizbarkeit und Stimmungsschwankungen.

## Revendications

1. Utilisation d'une composition comprenant, en tant que principes actifs, un extrait de pollen cytosolique, hydrosoluble et/ou liposoluble, et/ou un extrait de pistils cytosolique, hydrosoluble et/ou liposoluble, fertilisé par du pollen, ledit pollen et/ou lesdits pollen et pistils étant principalement obtenus à partir de plantes appartenant à la famille des graminées *(Poacée),* éventuellement associés à de la gelée royale et à de la vitamine E pour la fabrication d'un médicament destiné à traiter des troubles liés aux variations hormonales normales chez les femmes à l'âge de la péri- et de la post-ménopause.

2. Utilisation selon la revendication 1, dans laquelle l'extrait de pollen et de pistils est PI 82 et l'extrait de pollen est GC FEM.

3. Utilisation selon la revendication 2, dans laquelle les principes actifs sont inclus dans le médicament en des quantités de façon à obtenir un dosage quotidien de 60 mg à 960 mg de PI 82 ; de 18 mg à 288 mg de GC FEM ; et éventuellement de 2 mg à 48 mg de gelée royale et de 5 mg à 80 mg de vitamine E.

4. Utilisation selon la revendication 3, dans laquelle les principes actifs sont inclus dans le médicament en des quantités de façon à obtenir un dosage quotidien de 60 mg à 480 mg de PI 82 ; de 20 mg à 140 mg de GC FEM ; et éventuellement de 2 mg à 30 mg de gelée royale et de 5 mg à 60 mg de vitamine E.

5. Utilisation selon la revendication 4, dans laquelle les principes actifs sont inclus dans le médicament en des quantités de façon à obtenir un dosage quotidien de 60 mg à 360 mg de PI 82 ; de 20 mg à 80 mg de GC FEM ; et éventuellement de 2 mg à 15 mg de gelée royale et de 10 mg à 40 mg de vitamine E.

6. Utilisation selon la revendication 5, dans laquelle les principes actifs sont inclus dans le médicament en des quantités de façon à obtenir un dosage quotidien de 240 mg de PI 82 ; de 72 mg de GC FEM ; et éventuellement de 12 mg de gelée royale et de 20 mg de vitamine E.

7. Utilisation selon l'une quelconque des revendications 1 à 6, dans laquelle le ou les extrait(s) comprennent une ou des substances choisies parmi les imitations de SOD, les flavonoïdes, les tannins, les polyphénols, les caroténoïdes, des traces de substances oestrogènes, les bioflavonoïdes naturels, les vitamines les enzymes et les oligoéléments.

8. Utilisation selon l'un quelconque des revendications 1 à 7, dans laquelle le dosage quotidien du médicament est administré en une dose unique ou en plusieurs doses, de 1 à 8 fois par jour.

9. Utilisation selon l'une quelconque des revendications 1 à 8, dans laquelle les troubles sont choisis parmi les bouffées de chaleur, les tendances à la transpiration, les palpitations, les douleurs musculaires, les maux de tête, les difficultés à uriner, l'incontinence due au stress, la dysphorie, la sécheresse vaginale et des muqueuses, l'arthralgie, la rétention d'eau, l'irritabilité et les sautes d'humeur.
